# EUROPÄISCHE PATENTSCHRIFT

(11) **EP 2 314 351 B1**
(45) Veröffentlichungstag und Bekanntmachung des Hinweises auf die Patenterteilung: **17.12.2014**
(21) Anmeldenummer: 10156987.9
(22) Anmeldetag: 19.03.2010
(51) Int. Cl.: A61Q 5/10, A61Q 5/06, A61K 8/97, A61K 8/92

(54) **Haarfärbemittel mit verbesserter Hautverträglickeit**
Hair dye agent with improved skin tolerance
Colorant pour cheveux ayant une tolérance de la peau améliorée

(30) Priorität: 06.05.2009 DE 102009002877
(43) Veröffentlichungstag der Anmeldung: 27.04.2011
(73) Patentinhaber: Henkel AG & Co. KGaA, 40589 Düsseldorf (DE)
(72) Erfinder: Kleen, Astrid, 20457, Hamburg (DE); Rath, Susanne, 20359, Hamburg (DE); Zirwen, Sabrina, 22089, Hamburg (DE); Prillwitz, Dörte, 22089, Hamburg (DE)

(56) Entgegenhaltungen:
- EP-A1- 1 604 640
- WO-A1-2006/125619
- DE-A1-102006 017 312
- US-A- 5 972 322
- US-A1- 2007 074 355
- US-B1- 6 264 930
- Rahn: "Cleomilk Product Spotlight", , März 2009 (2009-03), XP002629409, Gefunden im Internet: URL:http://www.rahn-group.com/file_uploads /bibliothek/k_228_Cosmetics/k_467_Spotligh t/2695_0_cleomilkspotlight_de.pdf [gefunden am 2011-03-21]
- Rahn: "Cleomilk Phyto-Milk for Moisture and Care", , Februar 2009 (2009-02), XP002629410, Gefunden im Internet: URL:http://www.kinetiktech.com/brochures/p df/rahn/CLEOMILK_Leaflet_EN.pdf [gefunden am 2011-03-21]

## Beschreibung

Gegenstand der Erfindung ist ein Mittel zur Farbveränderung von keratinischen Fasern, dadurch gekennzeichnet, dass es in einem kosmetischen Träger mindestens eine farbverändernde Komponente sowie mindestens ein spezielle Pflegekombination enthält. Die erfindungsgemäßen Mittel eignen sich insbesondere zur Reduktion der Haarschädigung und zur Verbesserung der Hautverträglichkeit. Weiterhin betrifft die Erfindung ein Verfahren zur Anwendung solcher Mittel auf keratinhaltigen Fasern. Darüber hinaus betrifft die Erfindung die Verwendung des Mittels zur Färbung mit verringertem Irritationspotential.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe und Formleistung möglichst minimale Schädigungen auf dem Haar hervorrufen und insbesondere eine gute Hautverträglichkeit aufweisen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich zwar durch hervorragende, lang anhaltende Färbeergebnisse aus. Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese bilden dann im Rahmen oxidativer Prozesse im Haar naturanaloge Farbstoffe ausbilden.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Die dauerhafte Verformung von Keratinfasern wird üblicherweise so durchgeführt, dass man die Faser mechanisch verformt und die Verformung durch geeignete Hilfsmittel festlegt. Vor und/oder nach dieser Verformung behandelt man die Faser mit der wässrigen Zubereitung einer keratinreduzierenden Substanz, wobei diese einen Teil der Disulfid-Bindungen des Keratins zu Thiol-Gruppen, so dass es zu einer Lockerung der Peptidvernetzung spaltet und es infolge der Spannung der Faser durch die mechanische Verformung zu einer Neuorientierung des Keratingefüges kommt, und spült nach einer Einwirkungszeit mit Wasser oder einer wässrigen Lösung. In einem zweiten Schritt behandelt man dann die Faser mit der wässrigen Zubereitung eines Oxidationsmittels, unter dessen Einfluss erneut Disulfid-Bindungen geknüpft werden, und so das Keratingefüge in der vorgegebenen Verformung neu fixiert wird. Nach einer Einwirkungszeit wird auch dieses ausgespült und die Faser von den mechanischen Verformungshilfsmitteln (Wickler, Papilloten) befreit. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Färbemittel und Dauerwellmittel enthalten daher in der Regel Wasserstoffperoxid als Oxidationsmittel oder Fixiermittel. Dies führt neben dem gewünschten kosmetischen Effekt zu oxidativen Schädigungen an der Haarstruktur und zu einem erhöhten Irritationspotential der Kopfhaut. Auch der üblicherweise in solchen Mitteln basisch eingestellte pH-Wert führt zur Erhöhung des Irritationspotentials. Besonders Anwender mit empfindlicher Haut können auf solche Mittel mit der Ausbildung von Entzündungen und Schmerzgefühl reagieren. Daher sind solche Produkte insbesondere bei Personen mit Neigung zur Ausbildung von Allergien nur bedingt einsetzbar.

Aufgabe der vorliegenden Erfindung ist daher die Bereitstellung kosmetischer Mittel zur Farbveränderung keratinischer Fasern, die eine verbesserte Hautverträglichkeit aufweisen. Es ist insbesondere Aufgabe der vorliegenden Anmeldung, das Irritationspotential für die Haut zu senken und so eine Minimierung des Allergiepotentials zu erzielen.

In nicht vorhersehbarer Weise konnte nun gefunden werden, dass farbverändernde Mittel, die neben einer farbverändernden Komponente mindestens eine spezielle Pflegekombination, umfassend mindestens zwei pflanzlichen Ölen, wovon eines der Öle Sesamöl ist, und den Saft aus Blättern von Aloe Vera, enthalten, die oben genannten Nachteile vermeiden. Bedingt durch die Schutzwirkung bei Anwendung des erfindungsgemäßen Mittels kann die Hautverträglichkeit hierdurch verbessert.

Es hat sich gezeigt, dass diese Pflegekombination in Lage ist, in der Haut, insbesondere der Kopfhaut, die Reizschwelle der Haut, auf irritierende Substanzen zu reagieren, herabzusenken. Somit werden sowohl Entzündungspotential wie auch Schmerzempfindung der Kopfhaut reduziert. Außerdem kann sich angegriffene, empfindliche Haut beruhigen und so allergieauslösende Substanzen besser tolerieren. Schließlich wird die Haut zusätzlich durch ein verbessertes Feuchthaltevermögen und einen Rückfettungseffekt gepflegt. Weiterhin ist diese pflegekombination geeignet, die Haaroberfläche des behandelten Haares zu glätten und verleiht ihm damit einen verbesserten Glanz. Insbesondere führt die Verwendung dieser bestimmten Pflegekombination in Mittel zur Farbveränderung von menschlichen Haaren zu einer Verbesserung der Hautverträglichkeit und zu einer Verringerung an Irritationen auf der Kopfhaut.

Pflanzliche Pflegekombinationen basierend auf mindestens zwei pflanzlichen Ölen (Schwarzkümmelöl und Sesamöl) und dem Saft aus Blättern von Aloe Vera sind beispielsweise unter dem Handelsnamen Cleomilk bekannt.

Die WO 2006/125619 A1 offenbart farb- und formverändernde Mittel mit feuchtigkeitsregulierender und haarstrukturierender Wirkung, welche eine Wirkstoffkombination aus Alkyl- und/oder Alkenyl-Oligoglykosiden und dem Extrakt aus Aloe Vera enthalten.

In US 6 264 930 B1 wird eine warserhaltige Zusammenselzung zum Färben, Bleichen oder Konditionieren von Haaren enthaltend Wassserstoffperoxid, eine spezielle Silikonverbindung und Aloe Vera Extrakte offenbart.

Die US 2007/074355 A1 beschreibt Haarfärbemittel enthaltend Arginin zur Verstärkung der Haarquellung und ein Aloe Vera Gel.

In der EP 1 604 640 A1 werden Mittel zur Behandlung des Haares und der Kopfhaut enthaltend Fettsäureester eines Avocado Öls und verdickende Polymeren beschrieben, welche zusätzlich auch Sesamöl enthalten können.

DE 10 2006 017312 A1 beschreibt ein Herstellungsverfahren für Wasch- und Reinigungsmittel, welche als hautschülzenden Aktivstoff Sesamöl enthalten können.

Die US 5 972 322 A schließlich betrifft eine Mehrkomponenten-Verpackungseinheit zur individuellen Formulierung von speziell auf den Konsumenten abgestimmten Haarbehandlungsmitteln, welche Sesamöl oder Aloe Vera Öl als feuchtigkeitsspendende Wirkstoffe enthalten können.

Ein erster Gegenstand der Erfindung ist daher ein Mittel zur Farbveränderung von keratinischen Fasern, insbesondere menschlichen Haaren, enthaltend in einem kosmetischen Träger mindestens eine farb- und/oder formverändernde Komponente, dadurch **gekennzeichnet,** dass das Mittel zusätzlich eine Pflegekombination (PK) aus zwei pflegenden Komponenten i) und ii).
bestehend aus
i) einer Mischung von pflanzlichen Ölen, gewonnen aus einer Pflanzenart der Familie der Hahnenfußgewächse (Ranunculaceae) und aus Sesam (Sesamum Indicum), und
ii) dem Saft aus Blättern von Aloe Barbadensis (Aloe Vera),
enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als wesentlichen Inhaltsstoff enthält das erfindungsgemäße Mittel mindestens eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae) und aus Sesam (Sesamum Indicum), und
ii) dem Saft aus Blättern von Aloe Barbadensls (Aloe Vera).

Erfindungsgemäß wird als Öl einer Pflanzenart der Familie der Hahnenfußgewächse bevorzugt das Öl der Schwarzkümmelsamen (Nigella Sativa) verwendet, die kaltgepresst werden und nach der Filtration ein klares Öl liefern.

Eine Ausführungsform der vorliegenden Erfindung ist **dadurch gekennzeichnet, dass** im erfindungsgemäßen Mittel die Mischung von pflanzlichen Ölen als Öl, gewonnen aus einer Pflanzenart der Familie der Hahnenfußgewächse, das Öl aus Schwarzkümmel (Nigella Sativa) enthält.

Dieses Öl ist in der Naturheilkunde seit langem zur Linderung von Allergien, Neurodermitis und Schuppenflechte bekannt, denn es vermindert den transdermalen Wasserverlust und reguliert die Talgdrüsenfunktion. Schwarzkümmelöl umfasst einen hohen Anteil ungesättigter Fettsäuren (etwa 70% Palmitoleinsäure; Ölsäure, Linolsäure, Linolensäure) sowie weiterhin etherische Öle, Vitamine und Spurenelemente. Auf der Kopfhaut wirkt es besonders spröder, schuppiger Haut entgegen und macht sie wieder geschmeidig.

Schwarzkümmelöl wird in den erfindungsgemäßen Zusammensetzungen - bezogen auf deren Gesamtgewicht - in einer Menge von 0,000001 bis 3 Gew.-%, bevorzugt von 0,00001 bis 2 Gew.-%, mehr bevorzugt von 0,0001 bis 1 und insbesondere von 0,001 bis 0,5 Gew.-% eingesetzt.

Wie das Schwarzkümmelöl enthält auch das Sesamöl einen hohen Anteil ungesättigter Fettsäuren (Ölsäure und Linolsäure). Es wird aus den weißen und schwarzen Samen des Sesams (Sesamum Indicum) gewonnen und wird in der klassischen Hautkosmetik (beispielsweise in Hautcremes) wegen seiner hervorragenden rückfettenden Wirkung geschätzt.

Sesamöl wird in den erfindungsgemäßen Zusammensetzungen - bezogen auf deren Gesamtgewicht - in einer Menge von 0,00025 bis 10 Gew.-%, bevorzugt von 0,0005 bis 7 Gew.-%, mehr bevorzugt von 0,025 bis 5 Gew.-% und insbesondere von 0,05 bis 2,5 Gew.-%, eingesetzt.

Eine weitere Ausführungsform der vorliegenden Erfindung ist **dadurch gekennzeichnet, dass** im erfindungsgemäßen Mittel die Mischung der pflanzlichen Öle ein Gewichtsverhältnis von Sesamöl (a) zu dem Öl aus der Pflanzenart der Familie der Hahnenfußgewächse (b) mit einen Wert (a)/(b) zwischen 1000 bis 10, bevorzugt zwischen 500 bis 25, besitzt.

Neben den vorgenannten Ölen umfasst die Pflegekombination (PK) des erfindungsgemäßen Haarbehandlungsmittels weiterhin den Saft aus Blättern von Aloe Vera (Aloe Barbadensis).

Aloe Vera (Aloe Barbadensis) ist eine sukkulente Pflanze, deren Blättern aus drei unterschiedlichen Schichten bestehen, die jeweils spezifische Inhaltsstoffe enthalten. Nur die innerste Schicht Förderung bereits seit 2000 Jahren bekannt ist. Das Gel umfasst eine Vielzahl kosmetisch wertvoller Inhaltsstoffe, u.a. Vitamine (Vitamin A, Vitamin B1, Vitamin B2, Vitamin B3, Vitamin B6, Vitamin B9, Vitamin B12, Vitamin C und Vitamin E), Spurenelemente, Mineralstoffe, essentielle und nicht-essentielle Aminosäuren. Für kosmetische Zwecke wird dieser Saft auf schonende Weise konzentriert und/oder gefriergetrocknet und kann dem jeweiligen Mittel als Pulver zugesetzt werden. In der Vergangenheit wurde Aloe Vera Pulver überwiegend in der klassischen Hautkosmetik zur Behandlung und/oder Linderung von Abszessen, Akne, Fußpilz, Neurodermitis oder Herpes eingesetzt. In den erfindungsgemäßen Haarbehandlungsmitteln wirkt es stumpfen, glanzlosen Haaren und schuppiger, entzündeter Kopfhaut entgegen. Der gepulverte Saft der Blätter von Aloe Vera (Aloe Vera-Pulver) wird in den erfindungsgemäßen Zusammensetzungen - bezogen auf deren Gesamtgewicht - in einer Menge von 0,000001 bis 3 Gew.-%, bevorzugt von 0,00001 bis 2 Gew.-%, mehr bevorzugt von 0,0001 bis 1 und insbesondere von 0,001 bis 0,5 Gew.-% eingesetzt.

Es hat sich herausgestellt, dass die Pflegekombination insbesondere dann ihre positive Wirkung optimal entfalten, wenn im Mittel die pflanzlichen Öle und der gepulverte Saft aus Blättern von Aloe Vera in einem bestimmten Verhältnis zueinander stehen.

Eine weitere Ausführungsform der vorliegenden Erfindung ist daher ein Mittel, welches dadurch gekennzeichnet ist, dass im Mittel die Pflegekombination (PK) ein Gewichtsverhältnis zwischen der Mischung der pflanzlichen Öle i) und dem gepulverte Saft aus Blättern von Aloe Barbadensis (Aloe Vera) ii) mit einen Wert i)/ii) zwischen 1000 bis 10, bevorzugt zwischen 500 bis 25, besitzt.

Die vorgenannte Pflegekombination (PK) wird den erfindungsgemäßen Mitteln als Gemisch zugesetzt. Eine Ausführungsform der Erfindung ist daher dadurch gekennzeichnet, dass das Mittel die Pflegekombination (PK) in einer Menge von von 0,01 bis 3,0 Gew.-%, insbesondere von 0,05 bis 2,0 Gew.-%, jeweils bezogen auf das Gesamtgewicht des anwendungsbereiten Mittels, enthält.

Erfindungsgemäß als Pflegekombination (PK) geeignet ist beispielsweise der Einsatz des Handelsprodukts Cleomilk^{®} der Firma Rahn, das als pflanzliches Milchäquivalent in kosmetischen Hautbehandlungsmitteln vermarktet wird. Cleomilk® wird in den erfindungsgemäßen Mitteln - bezogen auf deren Gewicht - in Mengen von 0,001 bis 10 Gew.-%, bevorzugt in Mengen von 0,01 bis 7 Gew.-% und insbesondere in Mengen von 0,1 bis 5 Gew.-% eingesetzt.

Die erfindungsgemäßen Mittel werden zur Farbveränderung keratinischer Fasern eingesetzt. Erfindungsgemäß bevorzugte Mittel sind daher dadurch gekennzeichnet, das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens ein Aufhellmittel enthält.

In einer ersten Ausführungsform werden die farbverändernden Komponenten ausgewählt aus Oxidationsfarbstoffvorprodukten. Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Erfindungsgemäße Entwicklerkomponenten werden ausgewählt aus p-Phenylendiamin, zweikernigen Entwicklerkomponenten, p-Aminophenol, o-Aminophenol, heterocyclischen Entwickterkomponenten und/oder den Derivaten vorstehender Substanzklassen. Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxy-ethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]-tetramethylendiamin, N,N-Diethyl-N,N-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-di-aminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Amino-phenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethyl-phenol, 4-Amino-2-(2-hydroxyethoxy)phenol, 4-Amino-2-methylphenol, 4-Amino-2-hydroxy-methylphenol, 4-Amino-2-methoxymethyl-phenol, 4-Amino-2-aminomethylphenol, 4-Amino-2-[(2-hydroxyethyl)aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen der Formel (E3) sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazino-pyrazol, 1-Benzyl-4,5-diamino-3-methylpyrazol, 4,5-Diamino-3-t-butyhl-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze.

Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]-ethanol, 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxy-ethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxa-decan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-amino-phenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylamino-benzol, 3-Ethylamino-4-methylphenol, 2,4-Dichlor-3-aminophenol und den physiologisch verträglichen Salzen aller vorstehend genannten Verbindungen.

Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Di-aminophenoxy)ethanol, 1,3-Bis(2,4-diaminophenoxy)propan, 1-Methoxy-2-amino-4-(2'-hydroxy-ethylamino)benzol, 1,3-Bis(2,4-diaminophenyl)propan, 2,6-Bis(2'-hydroxyethylamino)-1-methyl-benzol, 2-({3-[(2-Hydroxyethyl)amino]-4-methoxy-5-methylphenyl}amino)ethanol, 2-({3-[(2-Hydroxy-ethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)aminobenzol und den physiologisch verträglichen Salzen der genannten Verbindungen.

Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen der genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxy-naphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxy-indolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)-benzol, 1,3-Bis-(2,4-diaminophenyl)propan, 2,6-Bis-(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Tri-hydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxy-indolin, 7-Hydroxyindolin oder Gemischen dieser Verbindungen oder den physiologisch verträglichen Salzen der vorgenannten Verbindungen.

Um das Irritationspotential der erfindungsgemäßen Mittel weiter zu senken, ist es erfindungsgemäß bevorzugt, die Oxidationsfarbstoffvorprodukte aus Farbstoffvorprodukten mit überwiegend geringem Irritationspotential auszuwählen. Insbesondere bevorzugt werden die Oxidationsfarbstoffvorprodukte daher ausgewählt aus der Gruppe, die gebildet wird aus 1,4-Diamino-2-(2-hydroxy-ethyl)benzol 1,5-Dihydroxynaphthalin, 2,7-Dihydroxynaphthalin, 4-Methylaminophenol, 2,4-Diaminophenoxyethanol Hydrochlorid, 3-Amino-2,4-dichlorphenol, 2-Methylresorcin, 2,4,5,6-Tetraaminopyrimidin, 1,3-Bis-(2,4-diaminophenoxy)propan, 3-Amino-2-chlor-6-methylphenol, 5-Amino-4-chlor-2-methylphenol Hydrochlorid, 2-Amino-3-hydroxypyridin, 2,8-Bis-(2-hydroxyethyl-amino)-1-methylbenzol Hydrochlorid, Bis-(2-hydroxy-5-aminophenyl)methan Hydrochlorid, N-(4-Amino-3-methylphenyl)-N-[3-(1H-imidazol-1-yl)propyl]amin.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel als farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitro-phenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphtho-chinon, Pikraminsäure und deren Salze, 2-Amino-6-chloro-4-nitrophenol, 4-Ethylamino-3-nitro-benzoesäure und 2-Chlor-6-ethylamino-4-nitrophenol.

Um das Irritationspotential der erfindungsgemäßen Mittel weiter zu senken, ist es erfindungsgemäß bevorzugt, die direktziehenden Farbstoffe aus Farbstoffen mit überwiegend geringem Irritationspotential auszuwählen. Insbesondere bevorzugt werden direktziehenden Farbstoffe daher ausgewählt aus der Gruppe, die gebildet wird aus Basic Brown 17, Basic Brown 16, Basic Violet 2, Basic Yellow 57, Basic Yellow 87, Basic Red 76, Basic Blue 99, Acid Orange 7, Acid Yellow 1, Acid Yellow 3, Acid Yellow 23, Acid Black 1, Acid Red 33, Acid Red 52, Acid Blue 9, HC Yellow 2, HC Yellow 13, HC Orange 1, HC Red 3, HC Red 7, HC Red 10, HC Red 11, HC Blue 2, HC Blue 12, Disperse Violet 1, N,N'-Bis-(2-hydroxyethyl)-2-nitro-1,4-diaminobenzol, N-(2-Hydroxyethyl)-2-nitro-4-methylanilin, 4-(3-Hydroxypropyl)amino-3-nitrophenol, 4-Amino-2'-carboxyl-2-nitro-diphenylamin.

In einer weiteren Ausführungsform der vorliegenden Erfindung wird die farbverändernde Komponente aus Farbstoffvorstufen naturanaloger Farbstoffe ausgewählt.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, insbesondere N-Methyl-5,6-dihydroxyindolin, N-Ethyl-5,6-dihydroxyindolin, N-Propyl-5,6-dihydroxyindolin, N-Butyl-5,6-dihydroxyindolin und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihy-droxyindol, 5,6-Dihydroxyindol-2-carbonsäure, vorzugsweise N-Methyl-5,6-dihydroxyindol, N-Ethyl-5,6-dihydroxyindol, N-Propyl-5,6-dihydroxyindol, N-Butyl-5,6-dihydroxyindol sowie insbesondere 5,6-Dihydroxyindöl.

Es ist nicht erforderlich, dass Oxidationsfarbstoffvorprodukte, direktziehende Farbstoffe oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

In einer weiteren Ausführungsform der vorliegenden Erfindung wird die farbverändernde Komponente aus Aufhellmitteln ausgewählt. Solche Aufhellmittel sind üblicherweise chemische Oxidationsmittel, mit deren Hilfe natürliche und künstliche Farbstoffe im Haar zerstört und das Haar damit gebleicht wird. Als Aufhellmittel kommen daher Peroxomonosulfate, Peroxodisulfate, Chlorite, Hypochlorite und insbesondere Wasserstoffperoxid oder dessen Anlagerungsprodukte an Harnstoff, Melamin sowie Natriumborat in Frage.

Der Aufhelleffekt kann auch neben einer Färbung gewünscht sein. Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Es ist daher bevorzugt, wenn das Mittel zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die Anlagerungsprodukte an Harnstoff, Melamin, Polyvinylpyrrolidinon sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und anorganischen Persulfaten eingesetzt, woraus eine Steigerung des Aufhellvermögens der Mittel resultiert. Bevorzugte Persulfatsalze sind Ammoniumperoxodisulfat, Kaliumperoxodisulfat und Natriumperoxodisulfat.

Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei die spezifische Pflegekombination (PK) in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten die spezifische Pflegekombination (PK).

Wird eine starke Aufhellung gewünscht, so ist es erfindungsgemäß bevorzugt, wenn zusätzlich eine Blondierzubereitung, enthaltend mindestens ein anorganisches, der Oxidationsmittelzubereitung vor Vermischung mit der erfindungsgemäßen Färbezubereitung beigemischt wird.

Weiterhin hat es sich als vorteilhaft erweisen, wenn die Oxidationsmittelzubereitungen aus Oxidationsfärbemittel und/oder Aufhellmitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriamin-(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetra-natriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäßen Mittel enthalten weitere Hilfs- und Zusatzstoffe.

Es hat sich herausgestellt, dass durch den Zusatz eines wasserlöslichen, organischen Lösungsmittels in das erfindungsgemäße Mittel die Haarschädigung weiter verringert.

Eine bevorzugte Ausführungsform der vorliegenden Erfindung ist daher dadurch gegeben, dass das Mittel weiterhin mindestens ein wasserlösliches, organisches Lösungsmittel enthält. Die organischen Lösungsmittel sind bei Raumtemperatur unter Normaldruck flüssig. Unter wasserlöslich wird hierin die Mischbarkeit in jedem Verhältnis unter Normalbedingungen verstanden. Bevorzugte organische Lösungsmittel sind dabei C₁-C₆-Alkohole, die gegebenenfalls weitere funktionelle Gruppen zur Verbesserung der Wasserlöslichkeit tragen, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether. Bevorzugte wasserlösliche, organische Lösungsmittel enthalten mindestens zwei Hydroxylgruppen. Bevorzugt sind diese ausgewählt aus der Gruppe, die gebildet wird aus 1,2-Ethandiol, 1,2-Propandiol (1,2-Propylenglykol), 1,3-Propandiol, 1,2-Butandiol, 1,3-Butandiol, 1,4-Butandiol, 2,3-Butandiol, 1,6-Hexandiol, 2-(2-Hydroxyethoxy)ethanol und Glycerin. Besonders bevorzugt ist dabei Glycerin.

Bevorzugt enthält das erfindungsgemäße Mittel wasserlösliche, organische Lösungsmittel zu 0,01 bis 10 Gew.-%, bevorzugt zu 0,1 bis 5 Gew.-%, jeweils bezogen auf das Gesamtgewicht des anwendungsbereiten Mittels.

Es hat sich weiterhin herausgestellt, dass sich die Hautirritation durch Haarfärbe mittel durch den Zusatz bestimmter Pflegestoffe zusätzlich unerwartet deutlich reduzieren lässt. Als besonders vorteilhaften Haarpflegestoff haben sich die Pflegestoffe, ausgewählt aus der Gruppe, gebildet aus Panthenol, Glycin, Serin, Arginin, Moringa Oleifera und/oder Ectoin, herausgestellt.

In einer bevorzugten Ausführungsform der Einfindung enthalten die Haarbehandlungsmittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% D-Panthenol.

In einer weiteren bevorzugten Ausführungsform der Einfindung enthalten die Haarbehandlungsmittel - bezogen auf ihr Gewicht - neben der erfindungsgemäßen Wirkstoffkombination 0,01 bis 2 Gew.-%, bevorzugt 0,05 bis 1,5 Gew.-% und insbesondere 0,1 bis 1 Gew.-% Glycin, Serin und/oder Arginin.

Als erfindungsgemäß besonders vorteilhaften zusätzlichen Pflegestoff hat sich weiterhin der Zellschutzstoff Ectoin ((*4S*)-1,4,5,6-Tetrahydro-2-methyl-pyrimidine-4-carboxylic acid; INCI-Bezeichnung: Ectoin) erwiesen.

In einer weiteren bevorzugten Ausführungsform der Einfindung enthalten die Haarbehandlungsmittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% Ectoin.

Erfindungsgemäß ganz besonders bevorzugt aufgrund ihrer haarkräftigenden Wirkung sowie ihrer unterstützenden Wirkung in Bezug auf die Haarfülle sind die Extrakte aus Grünem Tee, Kamille, Mandel und Moringa. Insbesondere bevorzugt ist erfindungsgemäß ein Moringa-Extrakt, beispielsweise ein unter dem Handelsnamen Puricare^{®} LS 9658 oder Moringa Oleifera^{®} vertriebenes Produkt.

In einer weiteren bevorzugten Ausführungsform der Einfindung enthalten die Haarbehandlungsmittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% Moringa-Extrakt.

Diese Pflegestoffe können einzeln, bevorzugt jedoch in Kombinationen untereinander im erfindungsgemäßen Mittel eingesetzt werden.

Durch den Zusatz oberflächenaktiver Verbindungen lässt sich die Effektivität der erfindungsgemäßen Mittel weiter verbessern. Als besonders vorteilhaft haben sich insbesondere oberflächenaktiver Verbindungen vom Typ nichtionischer Tenside herausgestellt, insbesondere Fettsäureester von Disacchariden.

Eine weitere Ausführungsform ist der vorliegenden Erfindung ist daher **dadurch gekennzeichnet,** dass das Mittel zusätzlich ein Acyl-Disaccharid enthält. Erfindungsgemäß vorteilhafte Acyl-Disaccharide sind insbesondere ausgewählt aus Saccharose Stearat (Sucrose Stearate), Trehalose Stearat, Cellobiose Stearat, Saccharose Palmitat, Trehalose Palmitat, Cellobiose Palmitat, Saccharose Oleat, Trehalose Oleat und Cellobiose Oleat, bevorzugt ist dabei Saccharose Stearat.

In einer weiteren bevorzugten Ausführungsform der Einfindung enthalten die Haarbehandlungsmittel - bezogen auf ihr Gewicht - neben der erfindungsgemäßen Wirkstoffkombination weiterhin 0,01 bis 2 Gew.-%, bevorzugt 0,005 bis 1,5 Gew.-% und insbesondere 0,01 bis 1 Gew.-% an Acyl-Disaccharid.

Es ist erfindungsgemäß bevorzugt, dass das Mittel zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die festen Anlagerungsprodukte an Harnstoff, Melamin, Polyvinylpyrrolidinon sowie Natriumborat in Frage.

Hinsichtlich der Mengen an Wasserstoffperoxid im anwendungsbereiten Mittel wird auf die voranstehenden, je nach Anwendungsgebiet bevorzugten Mengen verwiesen.

Vorzugsweise stellen die anwendungsbereiten Mittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und weiteren nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe.

Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Der Zusatz im erfindungsgemäßen Mittel bewirkt eine hervorragende Avivage, insbesondere eine verbesserte Nasskämmbarkeit.

Durch den Zusatz oberflächenaktiver Verbindungen lässt sich die Effektivität der erfindungsgemäßen Mittel weiter verbessern. Als besonders vorteilhaft haben sich insbesondere weitere oberflächenaktiver Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30 sowie Alkylpolyglykoside entsprechend der allgemeinen Formel RO-(Z)ₓ, wobei R für Alkyl, Z für Zucker sowie x für die Anzahl der Zuckereinheiten steht. Erfindungsgemäß bevorzugte Mittel enthalten als nichtionisches Tensid das Anlagerungsprodukt von Cocos-Fettalkohol an (Poly-)glucose, welches unter der INCI-Bezeichnung Coco-Glucoside bekannt ist.

Die anionischen, zwitterionischen, amphoteren und weiteren nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen.

Bevorzugt handelt es sich bei dem Verdickungsmittel um ein anionisches, synthetisches Polymer. Bevorzugte anionische Polymere sind Acrylsäure- und/oder Methacrylsäure-Polymerisate oder - Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogene Monomereinheiten bevorzugt Acrylamid, Methacrylamid, Acrylsäure-C₁-C₄-Alkylester und Methacrylsäure-C₁-C₄-Alkylester eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.
Es hat sich zur weiteren Verringerung des Irritationspotentials der erfindungsgemäßen Mittel als vorteilhaft herausgestellt, wenn die Mittel frei sind von organischen UV-Lichtschutzfiltersubstanzen. Unter UV-Lichtschutzfiltern sind organische Substanzen zu verstehen, die in der Lage sind, ultraviolette Strahlen zu absorbieren und die aufgenommene Energie in Form längerwelliger Strahlung, z. B. Wärme wieder abzugeben. Bevorzugt enthält das erfindungsgemäße Mittel keine UV-Filtersubstanzen aus den folgenden Substanzklassen: 3-Benzylidencampher und dessen Derivate, z. B. 3-(4-Methylbenzyliden)campher; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene); Ester der Salicylsäure, vorzugsweise Salicylsäure-2-ethylhexylester, Salicylsäure-4-isopropylben-zylester, Salicylsäurehomomenthylester; Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxy-benzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyltriazon; Propan-1,3-dione, wie z.B. 1-(4-tert-Butylphenyl)-3-(4'-methoxyphenyl)propan-1,3-dion oder 1-Phenyl-3-(4'-isopropylphenyl)-propan-1,3-dion; 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sul-fonsäure und ihre Salze; Sulfonsäurederivate des 3-Benzyliden-camphers, wie z.B. 4-(2-Oxo-3-bornylidenmethyl)benzolsulfonsäure und 2-Methyl-5-(2-oxo-3-bornyliden)sulfonsäure und deren Salze. In einer bevorzugten Ausführungsform der vorliegenden Erfindung die erfindungsgemäßen Mittel frei von UV-Lichtschutzfiltern, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenzimidazol-5-sulfonsäuren, Benzophenonsulfonsäuren und Benzoylmethanderivaten. Frei von UV-Lichtschutzfiltem im Sinne der vorliegenden Erfindung bedeutet, dass die anwendungsbereiten Mittel weniger als 0,05 Gew.-%, bevorzugt weniger als 0,005 Gew.-% und ganz besonders bevorzugt weniger als 0,0001 Gew.-% der UV-Lichtschutzfilter, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenz-imidazol-5-sulfonsäuren, Benzophenonsulfonsäuren und Benzoylmethanderivaten, enthalten.

Weiterhin hat es sich im Hinblick auf Reduktion der Hautirritation als vorteilhaft herausgestellt, wenn das erfindungsgemäße Mittel frei von Konservierungsmitteln auf Paraben-Basis ist. Parabene sind dem Fachmann als Konservierungsmittel für Lebensmittel wie auch für kosmetische Produkte bekannt. Unter Konservierungsmitteln auf Paraben-Basis sind im Sinne der vorliegenden Anmeldung die verzweigten und unverzweigten C₁-C₁₀-Alkylester der 4-Hydroxybenzoesäure sowie deren physiologisch verträglichen Salze, die sogenannten 4-Hydroxybenzoate, zu verstehen. Insbesondere bevorzugt sind die erfindungsgemäßen Mittel frei von Methyl-4-hydroxybenzoat (Methyl Paraben), Ethyl-4-hydroxybenzoat (Ethyl Paraben), Propyl-4-hydroxybenzoat (Propyl Paraben), 2-Propyl-4-hydroxybenzoat (Isopropyl Paraben), Butyl-4-hydroxybenzoat (Butyl Paraben), 2-Butyl-4-hydroxybenzoat (Isobutyl Paraben) und n-Heptyl-4-hydroxybenzoat (Heptyl Paraben) sowie den Natrium- und/oder Kaliumsalzen von Methyl-4-hydroxybenzoat. In einer bevorzugten Ausführungsform sind die erfindungsgemäßen Mittel daher frei von Konservierungsmitteln auf Paraben-Basis. Frei von Konservierungsmitteln auf Paraben-Basis im Sinne der vorliegenden Erfindung bedeutet, dass die anwendungsbereiten Mittel weniger als 0,005 Gew.-%, bevorzugt weniger als 0,0005 Gew.-% und ganz besonders bevorzugt weniger als 0,00001 Gew.-% der Parabene, ausgewählt aus der Gruppe, die gebildet wird aus den verzweigten und unverzweigten C₁-C₁₀-Alkylestern der 4-Hydroxybenzoesäure sowie deren physiologisch verträglichen Salzen, enthält.

Erfindungsgemäß ganz besonders bevorzugte Mittel sind frei von den oben genannten UV-Lichtschutzfiltern und/oder frei von Konservierungsmitteln auf Paraben-Basis.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und - distearat sowie PEG-3-distearat, Pigmente, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur Farbveränderung der Haare sind **dadurch gekennzeichnet, dass** sie einen möglichst neutralen pH-Wert besitzen. Eine weitere bevorzugte Ausführungsform der vorliegenden Erfindung besteht darin, dass das anwendungsbereite Mittel einen pH-Wert zwischen 4,0 und 12,0, bevorzugt zwischen 5,0 und 11,5, insbesondere bevorzugt zwischen 6,0 und 11,0 besitzt. Bei den pH-Werten im Sinne der vorliegenden Erfindung handelt es sich um pH-Werte, die bei einer Temperatur von 22 °C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (Monoethanolamin), Triethanolamin, Ammoniak, 1-Aminopropan-2-ol und 2-Amino-2-methylpropan-1-ol.

Die Konfektionierung der erfindungsgemäßen Farbveränderungsmittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen als vorteilhaft erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der efindungsgemäßen Komponenten separat verpackt wird; es kann aber bevorzugt sein, die Zubereitung, die die spezielle Pflegekombination (PK) enthält, bis zur Anwendung separat zu verpacken.

Erfindungsgemäß bevorzugte Mittel sind **dadurch gekennzeichnet, dass** das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel, welches in einem kosmetischen Träger mindestens ein Oxidationsfarbstoffvorprodukt und mindestens eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae) und aus Sesam (Sesamum Indicum), und
ii) dem Saft aus Blättern von Aloe Barbadensis (Aloe Vera), enthält und einen pH-Wert zwischen 5,0 und 12,0, bevorzugt zwischen 6,0 und 11,0 besitzt, enthält, und ein weiterer Container eine Oxidationsmittelzubereitung, enthaltend mindestens ein Oxidationsmittel, enthält.

Ein weiterer Gegenstand der vorliegenden Erfindung ist ein Verfahren zur Farbveränderüng keratinischer Fasern, bei dem ein erfindungsgemäßes Mittel gemäß obiger Vorgaben auf das Haar aufgetragen wird, für eine Einwirkzeit von 2 bis 45 Minuten, bevorzugt von 15 bis 30 Minuten auf dem Haar belassen wird, und anschließend das Haar ausgespült wird.

Eine bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt und mindestens und mindestens eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae) bevorzugt Schwarzkümmel, und aus Sesam (Sesamum Indicum), und
ii) dem Saft aus Blättern von Aloe Barbadensis (Aloe Vera), enthält, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 45 Minuten, bevorzugt von 15 bis 30 Minuten auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45 °C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt voneinander konfektionierte Container, wobei ein Container mindestens ein Container eine farbverändernde Verbindung und eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae), bevorzugt Schwarzkümmel, und aus Sesam (Sesamum Indicum), und
ii) dem Saft aus Blättern von Aloe Barbadensis (Aloe Vera), bevorzugt Schwarzkümmel, enthält und ein Container eine Oxidationsmittelzusammensetzung, enthaltend mindestens ein chemisches Oxidationsmittel, insbesondere Wasserstoffperoxid, enthält.

Eine bevorzugte Ausführungsform dieses Gegenstand der vorliegenden Erfindung ist eine Mehrkomponentenverpackungseinheit (Kit-of-Parts), enthaltend mindestens zwei getrennt voneinander konfektionierte Container, wobei ein Container eine Färbemischung in einem kosmetischen Träger, enthaltend mindestens eine farbgebende Komponente, insbesondere mindestens ein Oxidationsfarbstoffvorprodukt, und mindestens eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae), bevorzugt Schwarzkümmel, und aus Sesam (Sesamum Indicum), und
ii) dem Saft aus Blättern von Aloe Barbadensis (Aloe Vera), und ein Container eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung eines Mittels des ersten Erfindugsgegenstendes zur Steigerung des Hearglanzes bei der Farbveränderung menschlicher Haare.

Bezüglich weiterer bevorzugter Ausführungsformen der erfindungsgemäßen Verwendungen gilt mutatis mulandis das zu den erfindungsgemäßen Mitteln Gesagte.

Die nachfolgenden Beispiele sollen die vorliegende Erfindung verdeutlichen.

### Beispiele

### 1) Färbecremes

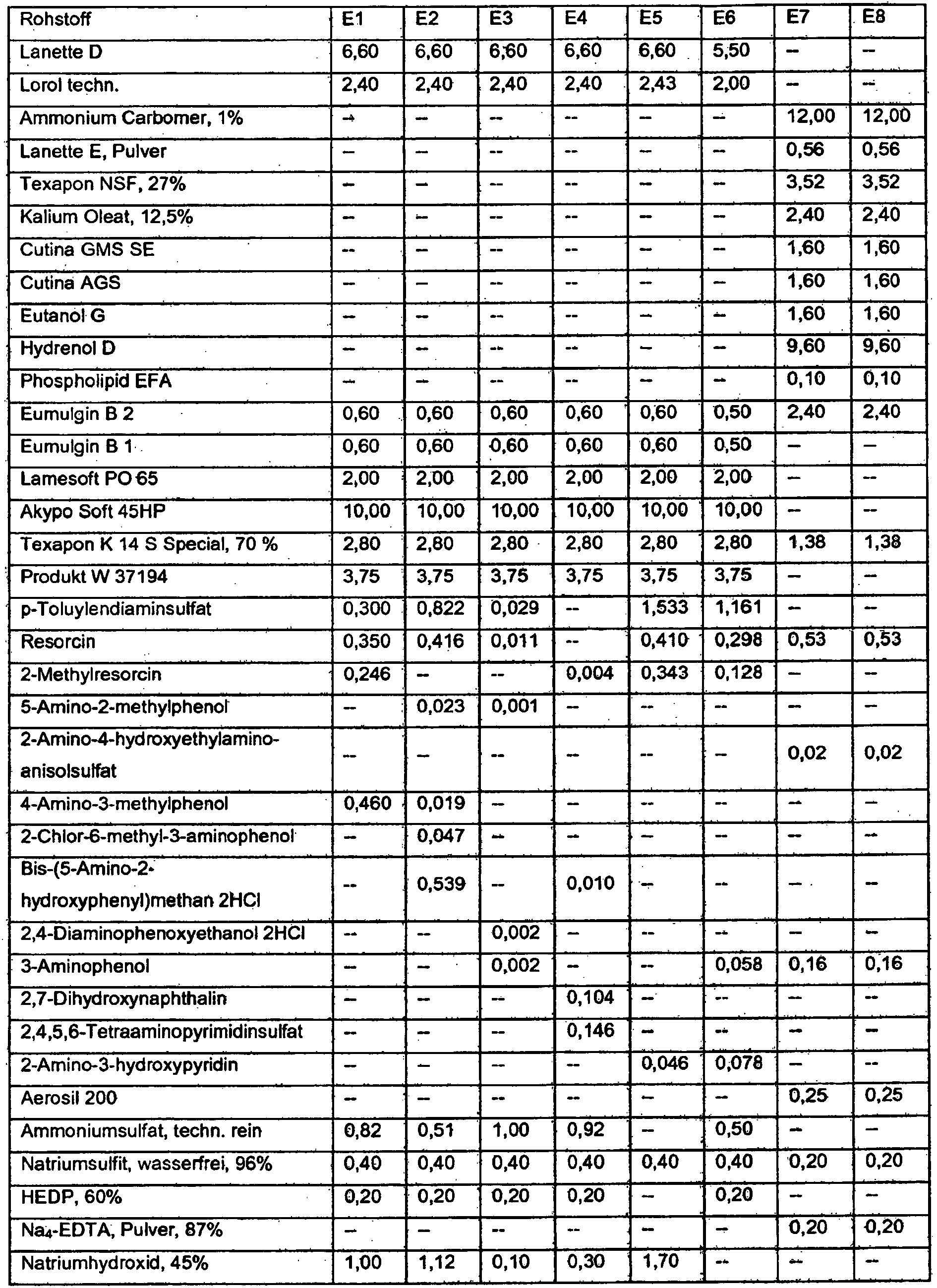

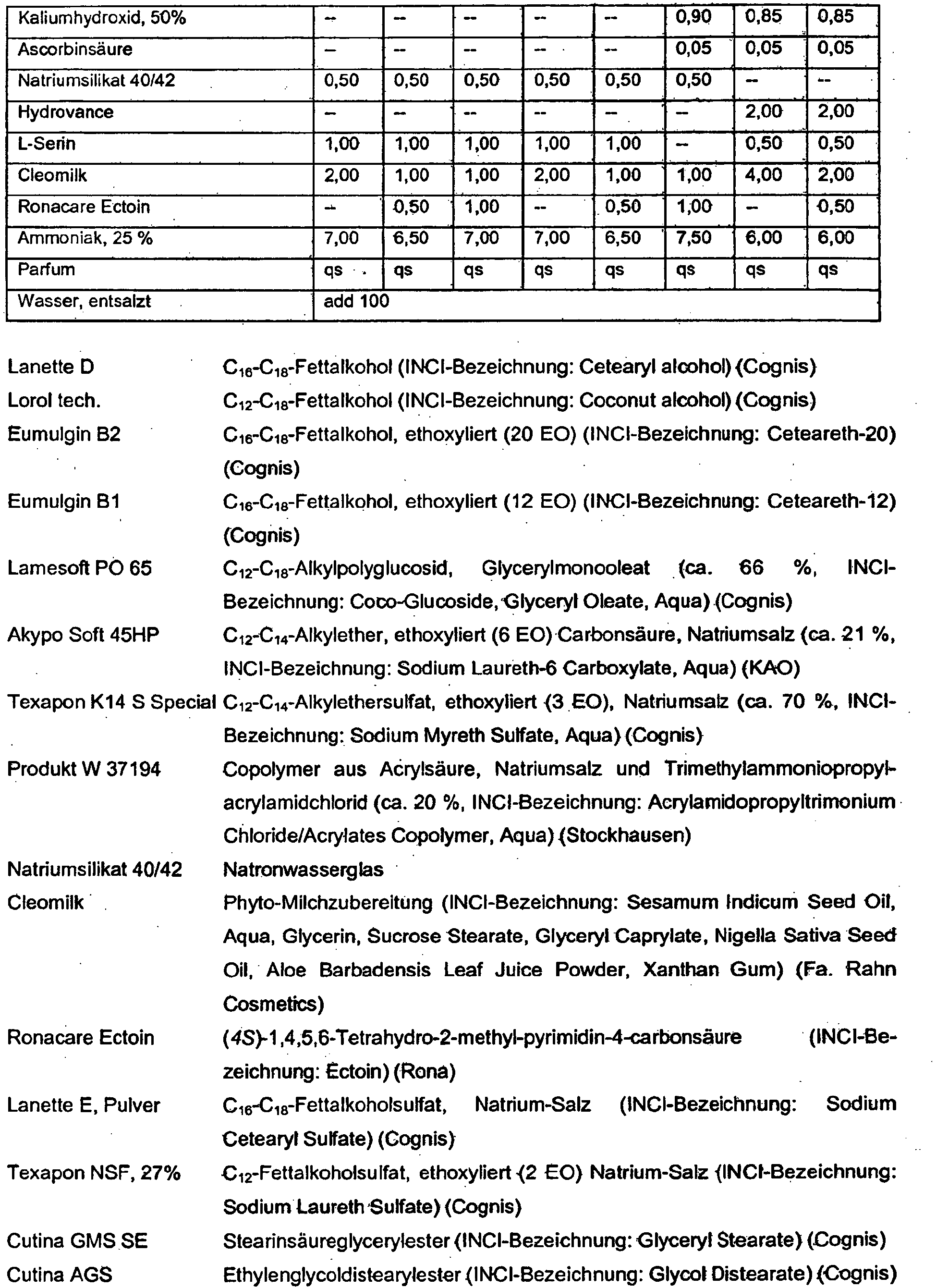

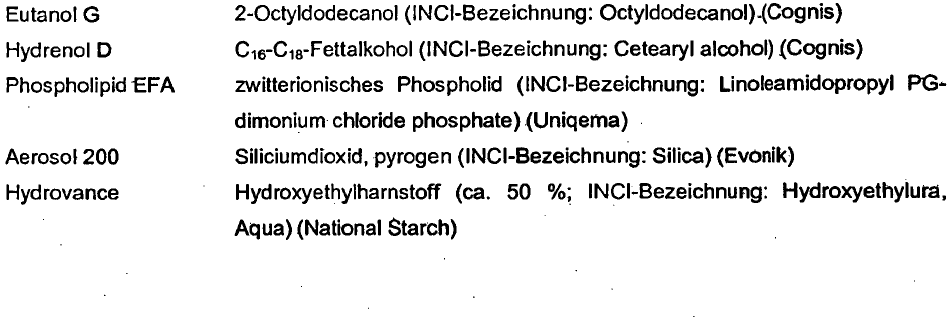

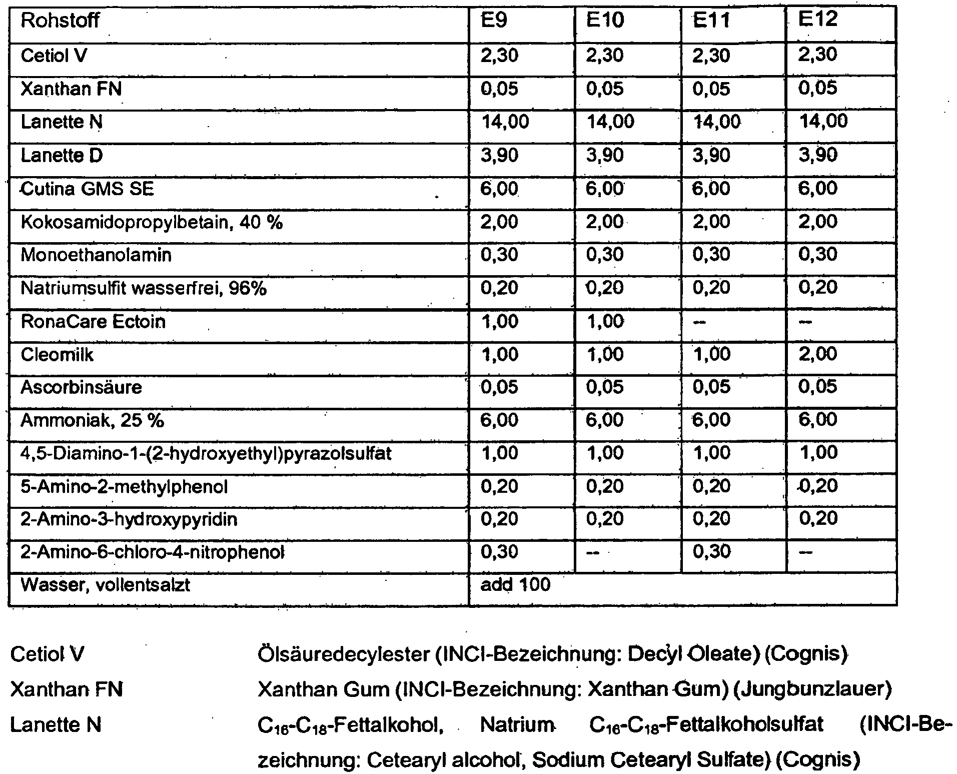

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.
**EW1:**

| Rohstoff | Gew.-% |
|---|---|
| Dipicolinsäure | 0,10 |
| Dinatriumpyrophosphat | 0,03 |
| HEDP, 60% | 1,50 |
| Texapon NSO | 2,00 |
| Dow Corning DB 110 A | 0,07 |
| Aculyn 33A | 15,00 |
| Ammoniak, 25 % | 0,65 |
| Wasser, vollentsalzt | ad 100 |

**EW2:**

| Rohstoff | Gew.-% |
|---|---|
| Na-benzoat | 0,04 |
| Dipicolinsäure | 0,10 |
| Dinatriumpyrophosphat | 0,19 |
| 1,2-Propandiol | 0,50 |
| HEDP, 60% | 0,25 |
| Paraffinum Liquidum | 0,30 |
| Genamin STAC | 0,20 |
| Cetearyl Alcohol | 3,00 |
| Eumulgin B 2 | 0,70 |
| Wasserstoffperoxid 50 % | 12,2 |
| Kaliumhydroxid, 50 % | 0,19 |
| Wasser | ad 100 |

- Texapon NSO UP: Laurylalkohol-diglykolethersulfat, Na-Salz (ca. 28%, INCl-Bezeichnung: Sodium Laureth Sulfate) (Cognis)
- Aculyn 33: Acrylpolymer (ca. 28% in Wasser; INCI-Bezeichnung: Acrylates Copolymer) (Rohm & Haas)
- Dow Corning DB 110: nicht ionische Silikonemulsion (INCI-Bezeichnung: Dimethicon) (Dow Coming)
- Genamin STAC: Trimethylstearylammonium chlorid (ca. 80% Aktivsubstanz; INCI-Bezeichnung: Steartrimonium Chloride) (Clariant)

### 3) Ausfärbungen:

Die Färbecremes E1 bis E6 wurden vor der Anwendung mit einer Entwicklerlösung EW1 im Gewichtsverhältnis von 1:1 versetzt und innig vermischt. Die Färbecremes E7 und E8 wurden vor der Anwendung mit einer Entwicklerlösung EW2 im Gewichtsverhältnis von 1:1 versetzt und innig vermischt. Die Färbecremes E9 bis E12 wurden vor der Anwendung mit einer 6 Gew.-%igen Wasserstoffperoxidzubereitung im Gewichtsverhältnis von 1:1 bis 1:2 versetzt und innig vermischt. Pro Gramm Haar (europäisches Humanhaar, Alkinco 6634, #10/2003, A9) wurden 4 g des frisch hergestellten, anwendungsbereiten Färbemittels aufgetragen. Die Einwirkzeit betrug 30 min bei 35°C für die Färbemittel. Danach wurden die Strähnen 30 s lang mit warmem Wasser ausgespült und luftgetrocknet. Die gefärbten Strähnen zeichneten sich durch glänzende Farben und einen angenehmen Griff aus.

## Patentansprüche

1. Mittel zur Farbveränderung von keratinischen Fasern, insbesondere menschlichen Haaren, enthaltend in einem kosmetischen Träger mindestens eine farbverändernde Komponente, **dadurch gekennzeichnet, dass** das Mittel zusätzlich eine Pflegekombination (PK) aus zwei pflegenden Komponenten i) und ii), bestehend aus
i) einer Mischung von pflanzlichen Ölen, gewonnen aus einer Pflanzenart der Familie der Hahnenfußgewächse (Ranunculaceae) und aus Sesam (Sesamum Indicum), und
ii) dem Saft aus Blättern von Aloe Barbadensis (Aloe Vera),
enthält.

2. Mittel nach Anspruch 1, **dadurch gekennzeichnet, dass** die Mischung der pflanzlichen Öle als Öl, gewonnen aus einer Pflanzenart der Familie der Hahnenfußgewächse, das Öl aus Schwarzkümmel (Nigella Sativa) enthält.

3. Mittel nach Anspruch 1 oder 2, **dadurch gekennzeichnet, dass** die Mischung der pflanzlichen Öle ein Gewichtsverhältnis von Sesamöl (a) zu dem Öl aus der Pflanzenart der Familie der Hahnenfußgewächse (b) mit einen Wert (a)/(b) zwischen 1000 bis 10, bevorzugt zwischen 500 bis 25, besitzt.

4. Mittel nach einem der Ansprüche 1 bis 3, **dadurch gekennzeichnet, dass** die Pflegekombination (PK) ein Gewichtsverhältnis zwischen der Mischung der pflanzlichen Öle i) und dem gepulverte Saft aus Blättern von Aloe Barbadensis (Aloe Vera) ii) mit einen Wert i)/ii) zwischen 1000 bis 10, bevorzugt zwischen 500 bis 25, besitzt.

5. Mittel nach einem der Ansprüche 1 bis 4, **dadurch gekennzeichnet, dass** es die Pflegekombination (PK) in einer Menge von 0,01 bis 3,0 Gew.-%, insbesondere von 0,05 bis 2,0 Gew.-%, jeweils bezogen auf das Gesamtgewicht des anwendungsbereiten Mittels, enthält.

6. Mittel nach einem der Ansprüche 1 bis 5, **dadurch gekennzeichnet, dass** das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens ein Aufhellmittel enthält.

7. Mittel nach einem der Ansprüche 1 bis 6, **dadurch gekennzeichnet, dass** das Mittel zusätzlich einen wasserlösliches, organisches Lösungsmittel mit mindestens zwei Hydroxylguppen, ausgewählt aus der Gruppe, umfassend 1,2-Ethandiol, 1,2-Propandiol, 1,3-Propandiol, 1,2-Butandiol, 1,3-Butandiol, 1,4-Butandiol, 2,3-Butandiol, 1,6-Hexandiol, 2-(2-Hydroxyethoxy)ethanol und Glycerin, enthält.

8. Mittel nach einem der Ansprüche 1 bis 7, **dadurch gekennzeichnet, dass** das Mittel zusätzlich ein Acyl-Disaccharid, ausgewählt aus Saccharose Stearat, Trehalose Stearat, Cellobiose Stearat, Saccharose Palmitat, Trehalose Palmitat, Cellobiose Palmitat, Saccharose Oleat, Trehalose Oleat und Cellobiose Oleat, enthält.

9. Mittel nach einem der Ansprüche 1 bis 8, **dadurch gekennzeichnet, dass** das Mittel als zusätzfiche Komponente einen Haarpflegestoff, ausgewählt aus der Gruppe Panthenol, Keratinhydrolysat, Glycin, Serin, Arginin, Moringa Oleifera und/oder Ectoin, enthält.

10. Mittel nach einem der Ansprüche 1 bis 9, **dadurch gekennzeichnet, dass** das Mittel zusätzlich ein Oxidationsmittel, ausgewählt aus Wasserstoffperoxid sowie seinen festen Anlagerungsprodukten an organische und anorganische Verbindungen, enthält.

11. Mittel nach einem der Ansprüche 1 bis 10, **dadurch gekennzeichnet, dass** es frei von UV-Filter-substanzen und/oder frei von Konservierungsmitteln auf Paraben-Basis ist.

12. Verwendung eines Mittels nach einem der Ansprüche 1 bis 11 zur Steigerung des Haarglanzes bei der Farbveränderung menschlicher Haare.

13. Mehrkomponentenverpackungseinheit (Kit-of-Parts), enthaltend mindestens zwei getrennt voneinander konfektionierte Container, wobei ein Container eine farbverändernde Verbindung gemäß einem der Ansprüche 1 bis 11 enthält und ein Container eine Oxidationsmittelzusammensetzung, enthaltend mindestens ein chemisches Oxidationsmittel, insbesondere Wasserstoffperoxid, enthält.

## Claims

1. An agent for modifying the colour of keratinic fibres, in particular human hair, containing in a cosmetic carrier at least one colour-modifying component, **characterised in that** the agent additionally contains a conditioning combination (CC) of two conditioning components i) and ii) consisting of
i) a mixture of plant oils obtained from a plant species from the buttercup family (*Ranunculaceae*) and from sesame (*Sesamum indicum*)*,* and
ii) the juice from *Aloe barbadensis* (*Aloe vera*) leaves.

2. An agent according to claim 1, **characterised in that** the mixture of plant oils contains the oil from black cumin (*Nigella sativa*) as the oil obtained from a plant species of the buttercup family.

3. An agent according to claim 1 or claim 2, **characterised in that** the mixture of plant oils has a weight ratio of sesame oil (a) to the oil from the plant species of the buttercup family (b) with a value (a)/(b) of between 1000-10, preferably between 500-25.

4. An agent according to one of claims 1 to 3, **characterised in that** the conditioning combination (CC) has a weight ratio between the mixture of plant oils i) and the powdered juice from *Aloe barbadensis* (*Aloe vera*) leaves ii) with a value i)/ii) of between 1000-10, preferably between 500-25.

5. An agent according to one of claims 1 to 4, **characterised in that** it contains the conditioning combination (CC) in a quantity of 0.01 to 3.0 wt.%, in particular of 0.05 to 2.0 wt.%, in each case relative to the total weight of the ready-to-use agent.

6. An agent according to one of claims 1 to 5, **characterised in that** the agent contains as colour-modifying component at least one oxidation dye precursor and/or at least one substantive dye and/or at least one precursor of nature-analogous dyes and/or at least one lightening agent.

7. An agent according to one of claims 1 to 6, **characterised in that** the agent additionally contains a water-soluble, organic solvent with at least two hydroxyl groups selected from the group comprising 1,2-ethanediol, 1,2-propanediol, 1,3-propanediol, 1,2-butanediol, 1,3-butanediol, 1,4-butanediol, 2,3-butanediol, 1,6-hexanediol, 2-(2-hydroxyethoxy)ethanol and glycerol.

8. An agent according to one of claims 1 to 7, **characterised in that** the agent additionally contains an acyl disaccharide selected from sucrose stearate, trehalose stearate, cellobiose stearate, sucrose palmitate, trehalose palmitate, cellobiose palmitate, sucrose oleate, trehalose oleate and cellobiose oleate.

9. An agent according to one of claims 1 to 8, **characterised in that** the agent contains as an additional component a hair conditioning substance selected from the group panthenol, keratin hydrolysate, glycine, serine, arginine, *Moringa oleifera* and/or ectoine.

10. An agent according to one of claims 1 to 9, **characterised in that** the agent additionally contains an oxidising agent selected from hydrogen peroxide and the solid addition products thereof onto organic and inorganic compounds.

11. An agent according to one of claims 1 to 10, **characterised in that** it contains no UV filter substances and/or contains no paraben-based preservatives.

12. Use of an agent according to one of claims 1 to 11 for increasing hair gloss on colour modification of human hair.

13. A multicomponent packaging unit (kit of parts) containing at least two containers packaged separately from one another, wherein one container contains a colour-modifying compound according to any one of claims 1 to 11 and one container contains an oxidising agent composition containing at least one chemical oxidising agent, in particular hydrogen peroxide.

## Revendications

1. Agent pour modifier la teinture de fibres kératiniques, en particulier de cheveux humains, contenant, dans un support cosmétique, au moins un composant modifiant la teinture, **caractérisé en ce que** l'agent contient en outre une combinaison de soins (PK) comprenant deux composants de soins i) et ii), constitués par
i) un mélange d'huiles végétales que l'on obtient à partir d'une espèce végétale de la famille des renonculacées (Ranunculaceae) et de sésame (Sesamum indicum) ; et
ii) la sève de feuilles de Aloe barbadensis (Aloe vera).

2. Agent selon la revendication 1, **caractérisé en ce que** le mélange des huiles végétales contient, à titre d'huile que l'on obtient à partir d'une espèce végétale de la famille des renonculacées, de l'huile de cumin noir (Nigella sativa).

3. Agent selon la revendication 1 ou 2, **caractérisé en ce que** le mélange des huiles végétales possède un rapport pondéral de l'huile de sésame (a) à l'huile de l'espèce végétale de la famille des renonculacées (b) avec une valeur (a)/(b) entre 1000 et 10, de préférence entre 500 et 25.

4. Agent selon l'une quelconque des revendications 1 à 3, **caractérisé en ce que** la combinaison de soins (PK) possède un rapport pondéral entre le mélange des huiles végétales i) et la sève réduite en poudre de feuilles de Aloe barbadensis (Aloe vera) ii) avec une valeur i)/ii) entre 1000 et 10, de préférence entre 500 et 25.

5. Agent selon l'une quelconque des revendications 1 à 4, **caractérisé en ce qu'**il contient la combinaison de soins (PK) en une quantité de 0,01 à 3,0 % en poids, en particulier de 0,05 à 2,0 % en poids, chaque fois rapportés au poids total de l'agent prêt à l'emploi.

6. Agent selon l'une quelconque des revendications 1 à 5, **caractérisé en ce que** l'agent contient à titre de composant modifiant la teinture, au moins un précurseur de colorant d'oxydation et/ou au moins un colorant direct et/ou au moins un précurseur de colorants analogues à ceux rencontrés dans la nature et/ou au moins un agent de décoloration.

7. Agent selon l'une quelconque des revendications 1 à 6, **caractérisé en ce que** l'agent contient en outre un solvant organique soluble dans l'eau comprenant au moins deux groupes hydroxyle, choisi parmi le groupe comprenant le 1,2-éthanediol, le 1,2-propanediol, le 1,3-propanediol, le 1,2-butanediol, le 1,3-butanediol, le 1,4-butanediol, le 2,3-butanediol, le 1,6-hexanediol, le 2-(2-hydroxyéthoxy)éthanol et le glycérol.

8. Agent selon l'une quelconque des revendications 1 à 7, **caractérisé en ce que** l'agent contient en outre un acyl-disaccharide choisi parmi le stéarate de saccharose, le stéarate de tréhalose, le stéarate de cellobiose, le palmitate de saccharose, le palmitate de tréhalose, le palmitate de cellobiose, l'oléate de saccharose, l'oléate de tréhalose et l'oléate de cellobiose.

9. Agent selon l'une quelconque des revendications 1 à 8, **caractérisé en ce que** l'agent contient, à titre de composant supplémentaire, une substance de soins capillaires choisie parmi le groupe comprenant le panthénol, l'hydrolysat de kératine, la glycine, la sérine, l'arginine, Moringa oleifera et/ou l'ectoïne.

10. Agent selon l'une quelconque des revendications 1 à 9, **caractérisé en ce que** l'agent contient en outre un agent d'oxydation choisi parmi le peroxyde d'hydrogène et ses produits solides de fixation par addition à des composés organiques et inorganiques.

11. Agent selon l'une quelconque des revendications 1 à 10, **caractérisé en ce qu'**il est exempt de substances filtrant les rayons ultraviolets et/ou exempt de conservateurs à base de parabènes.

12. Utilisation d'un agent selon l'une quelconque des revendications 1 à 11 pour augmenter le brillant capillaire lors d'une modification de la teinture de cheveux humains.

13. Unité de conditionnement à plusieurs composants (Kit-of-parts) contenant au moins deux récipients confectionnés pour être séparés l'un de l'autre, un récipient contenant un composé de modification de la teinture selon l'une quelconque des revendications 1 à 11 et un récipient contenant une composition d'agent d'oxydation contenant au moins un agent d'oxydation chimique en particulier du peroxyde d'hydrogène.
